# EUROPEAN PATENT APPLICATION

(11) **EP 1 661 908 A1**
(43) Date of publication of application: **31.05.2006**
(21) Application number: 04746176.9
(22) Date of filing: 21.06.2004
(51) Int. Cl.: C07K 4/12

(54) **REAGENT FOR AMPLIFYING AMYLOID FIBROSIS OF AMYLOID BETA-PROTEIN**

(30) Priority: 19.08.2003 JP 2003295153
(71) Applicant: Japan Science and Technology Agency, Kawaguchi-shi, Saitama 332-0012 (JP)
(72) Inventor: MIHARA, Hisakazu, 2530013 (JP); TAKAHASHI, Tsuyoshi, 2430036 (JP); OOSHIMA, Hideo, 2160003 (JP)
(74) Representative: Hiebl, Inge Elisabeth
(86) International application number: PCT/JP2004/008707
(87) International publication number: WO 2005/016957

(57) **Abstract**

There are disclosed a natural peptide search in which a template reaction with the nucleus of a minute amount of amyloid β-protein having undergone amyloid fibrosis is induced so as to form amyloid fibers, followed by fiber amount increase and amplification; designing and development of a novel artificial peptide which can be a substitute therefor; a method of amplifying the amyloid fibrosis of amyloid β-protein with the use thereof and a reagent for use therein; and a method of detecting disease caused by amyloidosis and a reagent for use therein. In particular, there are provided a method of amplifying the amyloid fibrosis of amyloid β-protein with the use of a reagent comprising a peptide composed of 14 to 23 residues of amyloid β-peptide or a peptide resulting from substitution of all the positive-charge side chain amino acids of the peptide with Lys and substitution of all the negative-charge side chain amino acids thereof with Glu; a reagent for use therein; a method of detecting disease caused by amyloidosis with the use of a reagent comprising the above peptide; a reagent for use therein; and a novel artificial peptide which can be used therein.

## Description

### Technical Field

The present invention relates to a method of amplifying amyloid β-protein (hereinafter, abbreviated as Aβ) having undergone amyloid fibrosis and a reagent therefor, which can be a basis for a method of diagnosis of signs of amyloidosis such as Alzheimer's disease and prion disease.

### Background Art

Alzheimer's disease is a disease reported in 1907 by a German neuropathologist Alois Alzheimer. This disease occurs in humans past middle age, and shows progressive dementia as a maj or symptom. Dementia is a state where originally normal intellectual functions are gradually degraded, thus resulting in obstacles in daily life. The first symptom of Alzheimer's disease is an obstacle in memory represented by failure of memory. Alzheimer's disease is further accompanied by obstacles in various intellectual functions including aphasia, agnosia and apraxia and is gradually developed to lead finally to severe states of dementia, such as bedridden state and incontinence. Pathologically, a large number of characteristic structures called senile plaques and changes in neurofibrils are observed in addition to diffuse cerebral shrinkage and loss of neurons. These changes tend to occur significantly in the hippocampus.

The changes in neurofibrils are those described for the first time by Alzheimer, and refer to accumulation of arygrophilic fibrous structures in the whole of neurons. Such fibrils are named paired helical filaments (PHF) because of their characteristic double helical structure. It was however found that PHF appears in many nerve diseases, and formation of PHF is considered at present as an unspecific reaction mode in the neuronal degenerative process.

Originally, the whole of a structure having amyloid fibrils gathering closely in the center and degenerating neuronal axons, astrocytes etc. gathering therearound has been defined as senile plaque. The amyloid fibrils are insoluble in various solvents, and by using this property, the fibrils are purified from meningeal blood vessels and cerebral parenchyma of Alzheimer's disease brain, and their constituent component was defined as a new peptide, amyloid β-protein (Aβ), having a molecular weight of about 4 kD. Because the molecular weight was low, the presence of its precursor was predicted, and APP (β-amyloid protein precursor) was identified by cDNA cloning. This precursor was once-transmembrane-type membrane protein, and Aβ was found to be a protein ranging from the extracellular domain of APP to two residues inside of the membrane domain.

Thereafter, an anti-Aβ antibody was prepared from the isolated Aβ, and when the brain of a patient with Alzheimer's disease was stained with the antibody, the presence of Aβ in many forms, besides the previously known globular senile plaques, was observed, and amyloid deposition was revealed to spread more widely than previously believed. Some amyloid deposits occur at light degrees and are not accompanied by degenerative nerve axons. This is named chronic senile plaque, considered as an initial pathological image of Alzheimer' s disease, and supported widely as being specific to Alzheimer's disease.

It is becoming evident in recent years that protein misfolding and amyloid fibrosis are important stages in lethal amyloidosis (disease where amyloid is deposited around cells or in gaps among tissues to cause functional obstacles) in Alzheimer' s disease and prion disease, and detection of amyloid fibrils is becoming very important in diagnosis of amyloidosis.

Aβ refers to peptides composed of 40 amino acid residues and 42 amino acid residues, which are called Aβ (1-40) and Aβ (1-42) respectively, and is considered to play an important role in the process of onset of Alzheimer's disease. Aβ is formed from once-transmembrane protein consisting of 695 to 770 residues by cleavage with enzymes (β-secretase, γ-secretase), and in the healthy human brain, the monomer Aβ (1-40) is estimated to occur at a concentration of about 0.1 nM to 10 nM, and is decomposed by α-secretase and metabolized.

Aβ (1-40) occurs in the form of a random coil under physiological conditions, and when Aβ (1-40) is formed, it is decomposed by α-secretase and metabolized without deposition in the brain. However, Aβ (1-42) is assembled under physiological conditions and converted from a random coil via an α-helix structure into a β-sheet structure. Thereafter, Aβ in the form of β-sheet structure is further polymerized to form amyloid fibrils to acquire protease resistance. Then, Aβ is insolublized and deposited in the brain to form senile plaques. This is considered to be important in the process of onset of Alzheimer's disease. However, Aβ (1-42) is scarcely formed, and the mechanism of forming Aβ (1-42) and the mechanism of forming amyloid fibrils therefrom has not been well elucidated.

In diagnosis of Alzheimer's disease, psychiatric techniques using DSM-4 (diagnostic criteria for Alzheimer's disease published by American Psychiatric Association) are mainly used in an early stage. Given the psychiatric techniques, however, the definite diagnosis of Alzheimer's disease in an early stage is difficult, and when a patient with Alzheimer's disease or his family comes to be aware of the disease, the morbidity has already proceeded considerably in many cases. The shrinkage of the brain in the middle stage or thereafter can be judged by MRI, but in a very early stage, the shrinkage of the brain is not initiated, thus making diagnosis of the disease difficult.

Other diagnostic methods make use of dilation of the pupil upon application of eye drops containing a low concentration of tropicamide (that is, an acetylcholine receptor antagonist to which the patient with Alzheimer' s disease is made sensitive because of a reduced level of acetylcholine receptors), a memory test by eye fixating (which is a test where damage to the hippocampus is examined because the hippocampus in the patient with Alzheimer's disease is significantly damaged) etc., but because of low reliability, these are used at present as mere secondary diagnostic methods. At present, the diagnosis of Alzheimer's disease in a very early stage is so difficult that treatment of Alzheimer's disease is made further difficult.

### Disclosure of the Invention

### Problem to be Solved by the Invention

Deposition of Aβ amyloid fibrils is specific to Alzheimer's disease, and occurs prior to other symptoms of Alzheimer's disease, so if Aβ formed into amyloid fibrils could be diagnosed in a very early stage of Alzheimer's disease, the definite diagnosis of Alzheimer' s disease would be feasible in the early stage. However, Aβ formed into amyloid fibrils in an early stage of Alzheimer' s disease occurs in a very small amount and is thus hardly detectable at present. Accordingly, if a very small amount of Aβ formed into amyloid fibrils could be amplified, such amplification would be promising in application to detection of Alzheimer's disease in an early stage.

The present invention has been made under these circumstances, and the object of the invention is to provide a natural peptide search in which a template reaction with the nucleus of a minute amount of Aβ having undergone amyloid fibrosis is caused to form amyloid fibrils, followed by fibril amount increase and amplification; design and development of a novel artificial peptide which can be a substitute therefor; a method of amplifying the amyloid fibrosis of amyloid β-protein by the use thereof and a reagent used therein; and a method of detecting disease attributable to amyloidosis and a reagent used therein. Means for Solving Problem

The present invention relates to a reagent for amplifying the amyloid fibrosis of amyloid β-protein, which includes a peptide consisting of 14 to 23 residues of amyloid β-peptide [hereinafter, abbreviated as Aβ (14-23)] or a peptide derived from the peptide by substituting all positively charged side-chain amino acids thereof with Lys and simultaneously substituting all negatively charged side-chain amino acids thereof with Glu.

The present invention also relates to a method of amplifying the amyloid fibrosis of amyloid β-protein, which includes using a reagent containing a peptide [Aβ (14-23)] consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

In addition, the present invention relates to a reagent for detection of disease attributable to amyloidosis, which includes a peptide [Aβ (14-23) consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

Further, the present invention relates to a method of detecting disease attributable to amyloidosis, which includes using a reagent containing a peptide [Aβ (14-23)] consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

Furthermore, the present invention relates to a peptide represented by the following general formula [1]:

R-Lys-Gln-Lys-Leu-Leu-X-Y-Leu-Glu-Glu-R' [1]

wherein R represents a hydrogen atom or an amino-protecting group, X represents Leu, Phe or Ala, Y represents Leu or Phe, and R' represents OH or NH₂.

### Effect of the Invention

According to the reagent for detection of diseases attributable to amyloidosis, the nucleus of amyloid fibrils occurring in the living body is amplified with a safe artificial peptide, whereby the undetectable causative protein can be amplified to a detectable level in a stage of omen, so diseases attributable to amyloidosis, such as Alzheimer's disease and prion disease (e.g. mad cow disease and human Creutzfeldt-Jakob disease) can be detected in an early stage.

### Brief Description of the Drawings

Fig. 1 shows a measurement result of a CD spectrum at 25°C of each peptide synthesized in Example 1. In Fig. 1, (1) shows a measurement result before incubation (= 0 day), and (2) shows a measurement result after incubation (1 day).
Fig. 2 shows time-dependent fluorescent spectral change (A) and time course (B) of fluorescent intensity at 482 nm of ThT in the presence of Aβ (10-35) [(1-2) in Example 2].
Fig. 3 shows a result of observation, under TEM, of the sample whose fluorescent intensity change was finished in (1-2) in Example 2.
Fig. 4 shows results of measurement and comparison of fluorescent intensity at 482 nmof ThT before and after incubation (room temperature) in the presence of each peptide according to the present invention, wherein D shows results before incubation, and ■ shows results after incubation [(1-3) in Example 2].
Fig. 5 shows results of measurement and comparison of fluorescent intensity at 482 nm of ThT before and after incubation (40°C) in the presence of each peptide according to the present invention, wherein D shows results before incubation, and ■ shows results after incubation [(1-3) in Example 2].
Fig. 6 shows a result of observation, under TEM, of the sample whose fluorescent intensity change was finished by incubation at 40°C in the presence of an artificial peptide (10-3L), in (1-3) in Example 2.
Fig. 7 shows results of measurement and comparison of fluorescent intensity at 482 nm of ThT in the presence of each peptide according to the present invention, with or without Aβ (10-35) (nucleus) formed into fibrils. In Fig. 7, D shows results without the nucleus (after 1 day) and ■ shows results with 10 µM nucleus (after 1 day) [(2) in Example 2].
Fig. 8 shows the degree of increase of fluorescent intensity at 490 nm of ThT in the presence of each peptide according to the present invention, with or without Aβ (10-35) (nucleus) formed into fibrils. In Fig. 8, □ shows results without Aβ (10-35) and ■ shows results with Aβ (10-35) [Example 3].

### Best Mode for carrying out the Invention

Aβ (14-23) used in the present invention may be a naturally occurring peptide or a peptide synthesized by a conventional method.

Preferable examples of the Aβ (14-23) peptide used in the present invention wherein all positively charged side-chain amino acids are substituted with Lys and simultaneously all negatively charged side-chain amino acids are substituted with Glu include, for example, peptides of Aβ (14-23) wherein all positively charged side-chain amino acids are substituted with Lys, all negatively charged side-chain amino acids are substituted with Glu, and one or more hydrophobic residues in a peptide chain are substituted with other hydrophobic amino acid residues. The other hydrophobic amino acid residues capable of substitution include, for example, amino acid residues such as leucine (Leu), isoleucine (Ile), phenylalanine (Phe) and valine (Val).

Preferable examples of such peptides include, but are not limited to, peptides derived from Aβ (14-23) by substituting all positively charged side-chain amino acids thereof with Lys and simultaneously substituting all negatively charged side-chain amino acids thereof with Glu, and further by i) substituting all hydrophobic residues in the peptide chain with Leu, ii) substituting all hydrophobic residues (excluding Phe at the position 3 or 4 from the N-terminal side of the hydrophobic site) in the peptide chain with Leu, or iii) substituting Ala at the position 3 from the N-terminal side of the hydrophobic site with an Ala and simultaneously substituting the remaining hydrophobic residues with Leu.

The reagent for amplifying the amyloid fibrosis of amyloid β-protein according to the present invention, and the reagent for detection of disease attributable to amyloidosis according to the present invention, are characterized by including the above-mentioned Aβ (14-23) or a peptide derived therefrom by substituting all positively charged side-chain amino acids thereof with Lys and simultaneously substituting all negatively charged side-chain amino acids thereof with Glu.

The method of amplifying the amyloid fibrosis of amyloid β-protein according to the present invention, and the method of detecting disease attributable to amyloidosis, are characterized by using a reagent including the Aβ (14-23) or a peptide derived therefrom by substituting all positively charged side-chain amino acids thereof with Lys and simultaneously substituting all negatively charged side-chain amino acids thereof with Glu.

Preferable examples of Aβ (14-23) wherein all positively charged side-chain amino acids are substituted with Lys and simultaneously all negatively charged side-chain amino acids are substituted with Glu are represented by, for example, the following general formula:

R-Lys-Gln-Lys-Leu-Leu-X-Y-Leu-Glu-Glu-R' [1]

wherein R represents a hydrogen atom or an amino-protecting group, X represents Leu, Phe or Ala, Y represents Leu or Phe, and R' represents OH or NH₂.

Preferable examples of the peptides represented by the general formula [1] above include, for example:
1) R-Lys-Gln-Lys-Leu-Leu-Leu-Leu-Leu-Glu-Glu-R'
2) R-Lys-Gln-Lys-Leu-Leu-Leu-Phe-Leu-Glu-Glu-R'
3) R-Lys-Gln-Lys-Leu-Leu-Phe-Leu-Leu-Glu-Glu-R'
4) R-Lys-Gln-Lys-Leu-Leu-Ala-Leu-Leu-Glu-Glu-R'

(R and R' have the same meanings as defined above.)

Any peptides shown in 1) to 4) above are novel compounds.

The amino-protecting group represented by R in the general formula [1] includes, for example, an acyl group such as an acetyl group, t-butoxycarbonyl group, benzyloxycarbonyl group, fluorenyl methoxy carbonyl group, benzoyl group etc., and a fluorescent acyl group such as a fluorescein group, Oregon green group etc., among which an easier acetyl group is preferable.

In the peptides shown in 1) to 4) above, R has the same meaning as defined above.

Preferable examples of the Aβ (14-23) peptide used in the present invention, wherein all positively charged side-chain amino acids are substituted with Lys and simultaneously all negatively charged side-chain amino acids are substituted with Glu, include not only the peptides represented by the general formula [1] above, but also peptides of the general formula [1]
wherein X is present at the first, second or fifth position from the N-terminal side of the hydrophobic site in the peptide chain, or peptides of the general formula [1] wherein Leu is substituted by Ile, or peptides of the general formula [1] wherein X and Y are substituted with Ile respectively.

Any of these peptides according to the present invention can be easily synthesized by conventional methods such as a Boc solid phase synthesis method or Fmoc solid phase method using a peptide synthesizer.

The disease which can be detected by the reagent for detection of disease attributable to amyloidosis or by the method for detection of disease attributable to amyloidosis, according to the present invention, is firstlyAlzheimer' s disease. Prion disease such as mad cow disease or Creutzfeldt-Jakob disease can also be similarly detected. Besides, various diseases attributable to amyloidosis can also be similarly detected.

These various diseases attributable to amyloidosis can be detected highly sensitively in a stage of omen or an early stage by using the detection reagent containing the peptide of the present invention.

Hereinafter, the present invention is described in more detail by reference to the Examples, but the present invention is not limited by these examples.

### Example 1

### (1) Synthesis of peptide Ac-Lys-Gln-Lys-Leu-Leu-Leu-Phe-Leu-Glu-Glu-NH₂ [peptide (10-4F)]

### (1-1) Reagents, instruments/devices

Fmoc amino acid derivatives, and resin as solid-phase carriers, were purchased from Novabiochem and used as they were. As other reagents, commercial products were used as they were.

The peptide was synthesized manually by an Fmoc (9-fluorenyl methoxycarbonyl) solid phase method. As a container for manual synthesis of the peptide chain, a polypropylene empty column (manufactured by Pharmacia Biotech) was used. An absorption spectrumwas measured by using SHIMADZU BioSpc-1600 Spectrometer. In high-performance liquid chromatography (HPLC), HITACHI 7000 system was used. A mixed solvent of 0.1% TFA/H₂O as solution A and 0.08% TFA/CH₃CN as solution B was used as HPLC solvent, and eluted with a linear gradient of solutions A and B for 30 minutes. As a reverse phase column, Cosmosil 5C18-AR-2 (Nacalai tesque) (4.6×150 mm) was used at a flow rate of 1 ml/min for analysis. For peptide purification, Cosmosil 5C₁₈-AR-2 (Nacalai tesque) (10×250 mm) was used at a flow rate of 3 ml/min. The detection wavelength 220 nm was used. In time-of-flight mass spectrometry (TOF-MS), SHIMADZU KRATOS MALDI III was used, and as matrix, 3,5-dimethoxy-4-hydroxycinnamic acid (sinapic acid) was used. A fluorescence plate reader, BERTHOLD Twinkle LB970 was used.

### (1-2) Synthesis of the peptide (10-4F)

Synthesis of 10-4F was carried out in the scale of 20 µmol by the Fmoc solid phase method. As the resin, NovaSyn TGR resin was used. In coupling of each amino acid residue, 3 equivalents of Fmoc-amino acid (Fmoc-AA-OH), relative to the amino group on the resin, were used so that the reaction proceeded completely. As Fmoc-AA-OH in the present example, Fmoc-Gln(Trt)-OH, Fmoc-Glu(Ot-Bu)-OH, Fmoc-Leu-OH, Fmoc-Lys (Boc)-OH, and Fmoc-Phe-OH (t-Bu, tert-butyl; Trt, trityl; Boc, tert-butoxycarboxyl) were used. As coupling reagents, 2-(1H-benzotriazol-1-yl)-1,1,3,3-tetramethyl uronium hexafluorophosphate (HBTU) (3 equivalents), 1-hydroxybenzotriazole hydrate (HOBt-H₂O) (3 equivalents) and N,N-diisopropyl ethylamine (DIEA) (6 equivalents) were used. As the reaction solvent, N-methyl pyrrolidone (NMP) was used. For removal of Fmoc group, 20% piperidine (PPD)/NMP was used. Successive coupling of each amino acid derivative with the carboxyl terminus was carried out. During the reaction, the reaction mixture was stirred suitably with a vortex, and the conclusion of the coupling was confirmed by a Kaiser Test (KT) . After the coupling of all amino acids, the Fmoc group of the amino acid in the amino terminus of the peptide was removed, and acetic anhydride (10 equivalents) in NMP was used in acetylation of the N-terminal amino acid of the peptide. Thereafter, the resin was washed successively with NMP and chloroform and then dried under reduced pressure for 4 hours.

The resin dried under reduced pressure was placed in a 50-ml Kjeldahl flask (eggplant type flask), and m-cresol (0.13 ml), thioanisole (0.38 ml) and trifluoroacetic acid (TFA) (5 ml) were added successively and reacted under stirring at roomtemperature for 1 hour. After TFA was removed by an evaporator, about 30 ml diethyl ether was added to precipitate the peptide (10-4F). The sample was subjected 5 times to centrifugation (3000 rpm, 5 minutes) and subsequent decantation, and then the diethyl ether was gasified in a nitrogen stream. The precipitates were dried under reduced pressure to give crude peptide (10-4F). The resulting crude peptide (10-4F) was purified by reverse phase HPLC, and after CH₃CN was removed by an evaporator, the product was lyophilized and recovered. The yield was 35%. The resulting peptide (10-4F) was identified by MALDI-TOFMS.

MALDI-TOFMS:
Found (M+H⁺) 1301.6
Calculated (M+H⁺) 1302.5

### (2) Synthesis of peptide Ac-Lys-Gln-Lys-Leu-Leu-Leu-Leu-Leu-Glu-Glu-NH₂ [peptide (10-3L)], peptide Ac-Lys-Gln-Lys-Leu-Leu-Phe-Leu-Leu-Glu-Glu-NH₂ [peptide (10-3F)] and peptide Ac-Lys-Gln-Lys-Leu-Leu-Ala-Leu-Leu-Glu-Glu-NH₂ [peptide (10-3A)]

Using the same reagents and instruments/devices as used in (1) above, peptide synthesis and purification by reverse phase HPLC were carried out in the same manner as in (1) above, to synthesize the peptides (10-3L), (10-3F) and (10-3A) respectively.

The MALDI-TOFMS of these resulting peptides is as follows.

| | |
|---|---|
| Peptide (10-3L): | Found (M+H⁺) 1290.1 |
| | Calculated (M+H⁺) 1290.6 |
| Peptide (10-3F): | Found (M+H⁺) 1302.2 |
| | Calculated (M+H⁺) 1302.5 |
| Peptide (10-3A): | Found (M+Na⁺) 1226.2 |
| | Calculated (M+Na⁺) 1226.5 |

### (3) Synthesis of Aβ (14-23) and Aβ (10-35)

Using the same reagents and instruments/devices as used in (1) above, peptide [Aβ (14-23)] consisting of 14 to 23 residues of amyloid β peptide, and peptide [Aβ (10-35)] consisting of 10 to 35 residues of amyloid β peptide, which as the nucleus of amyloid fibril, is known to be rich in hydrophobic residues and to have a high ability to form fibrils, were synthesized in the same manner as in (1) above.

The MALDI-TOFMS of these resulting peptides is as follows.

| | |
|---|---|
| Aβ (14-23): | Found (M+H⁺) 1274.8 |
| | Calculated (M+H⁺) 1274.4 |
| Aβ (10-35): | Found (M+H⁺) 2946.5 |
| | Calculated (M+H⁺) 2945.3 |

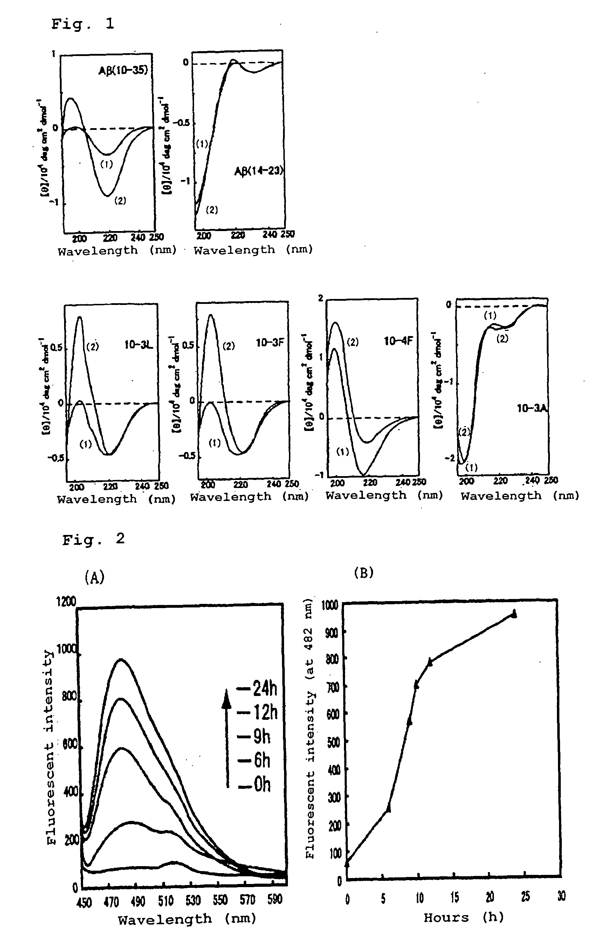

Each peptide synthesized in the present examples was measured for its CD spectrum at 25°C, and the result is shown in Fig. 1. In Fig. 1, (1) shows a measurement result before incubation (= 0 day), and (2) shows a measurement result after incubation (1 day).

### Example 2

### (1) Amyloid fibrosis by using the artificial peptide alone

The peptide (10-4F), (10-3L), (10-3F), (10-3A), Aβ (14-23) or Aβ (10-35) synthesized in Example 1, which was contained alone in an aqueous solution, was evaluated for its ability to form amyloid fibrils. The secondary structure was examined using a CD spectrum, and the ability to form amyloid fibrils was evaluated with dye thioflavin T (ThT) binding specifically to amyloid fibrils to emit fluorescence, under a transmission electron microscope (TEM).

### (1-1) Measurement of the secondary structure of each peptide by a CD spectrum

Solutions of the respective synthesized peptides, 2 mM, in trifluoroethanol (TFE) were prepared and used as stock solutions respectively. Each stock solution was diluted to a final peptide concentration of 100 µM with 20 mM Tris-HCl buffer (pH 7.4) , then incubated at ordinary temperatures, and measured for CD spectrum at 25°C (Fig. 1).

As a result, 10-4F, 10-3L, 10-3F and Aβ (10-35) had spectra unique to β-sheet structure and showing the negative maximum in the vicinity of 218 nm and the positive maximum in the vicinity of 205 nm from the start, and increased signals with time to increase β-sheet properties, thus suggesting the progress of amyloid fibrosis. Both 10-3A and Aβ (14-23) had spectra unique to random coil state and showing no spectral change with time.

Among these artificial peptides, the peptides into which Ala poor in hydrophobicity had been introduced were in the form of a random coil, while the other peptides had a β-sheet structure, thus suggesting that the hydrophobicity in the peptide chain was important for formation of amyloid fibrils.

### (1-2) Selection, with ThT, of conditions for forming amyloid fibrils of Aβ (10-35)

The formation of amyloid fibrils of Aβ (10-35) was monitored with ThT (λex = 440 nm, λem = 482 nm), to select conditions for incubation of Aβ (10-35) used as the nucleus of amyloid fibrils (Fig. 2).

### Incubation conditions

- Starting solution used: [Aβ (10-35)] = 2 mM solution in TFE
- Concentration: [Aβ (10-35)] = 100 µM in 20 mM Tris-HCl buffer (pH 7.4)
- Temperature: room temperature

### Measurement conditions

- Concentrations: [Aβ (10-35)] = 5 µM, [ThT] = 6 µM (20 mM Tris-HCl buffer (pH 7.4))
- Temperature: room temperature
- Stirring conditions: The whole volume of the reaction mixture was sufficiently stirred with a Pasteur pipette before sampling, and then sufficiently stirred in a cell with a Pasteur pipette.
- Excitation wavelength: 440 nm, detection wavelength: 482 nm.

### Results

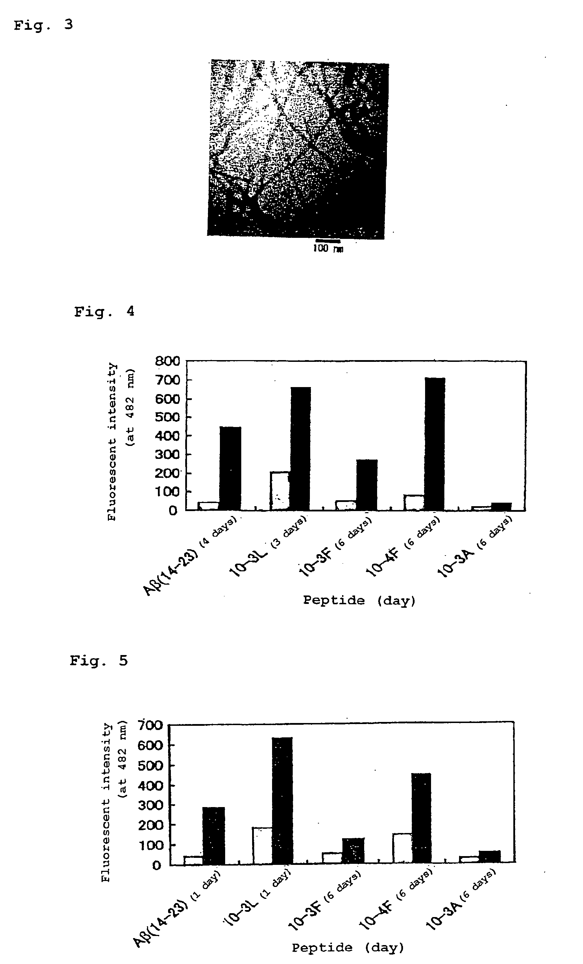

Aβ (10-35) increased fluorescent intensity in the vicinity of 482 nm in the presence of ThT with time, and showed a sigmoid change when the fluorescent intensity was plotted on a graph. This suggests autonomous replication and autonomous catalytic reaction characteristic of amyloid protein. When the sample whose fluorescent intensity change hadbeen finishedwas observed under TEM, fibrils were observed (Fig. 3).

The change was finished in about 24 to 30 hours, and the fluorescent intensity upon completion of formation of amyloid fibrils and the time having elapsed until the completion of the change were excellent in reproducibility, and thus a sample which is incubated as a nucleus under this condition for 30 hours or more, showed a fluorescent intensity of about 700 to 1000 in the presence of ThT, was used as the nucleus of Aβ.

### (1-3) Evaluation, with ThT, of the ability of the artificial peptide used alone to form amyloid fibrils

The ability of each artificial peptide obtained in Example 1 to form amyloid fibrils when used alone was evaluated using ThT.

### Incubation conditions

- Starting solution used: [peptide] = 2 mM solution in TFE
- Concentration: [peptide] = 200 µM in 20 mM Tris-HCl buffer (pH 7.4)
- Temperature: room temperature or 40°C

### Measurement conditions

- Concentrations: [peptide] = 5 µM, [ThT] = 6 µM (20 mM Tris-HCl buffer (pH 7.4))
- Temperature: room temperature
- Stirring conditions: The whole volume of the reaction mixture was sufficiently stirred with a Pasteur pipette before sampling, and then sufficiently stirred in a cell with a Pasteur pipette.
- Excitation wavelength: 440 nm, detection wavelength: 482 nm.

### Results

### (i) Case where incubation was carried out at room temperature (ordinary temperatures)

The artificial peptides 10-3L, 10-3F and 10-4F showed a significant increase in fluorescent intensity with time, thus suggesting that each artificial peptide used alone had an ability to form amyloid fibrils. The peptide 10-3A did not show an increase in fluorescent intensity. The time having elapsed until the increase in fluorescent intensity was finished, was shortest (= 3 days) in the case of 10-3L, or was about 6 days in the case of 10-3F and 10-4F. In the end point, 10-3L and 10-4F showed similar fluorescent intensity, while 10-3F showed fluorescent intensity which was half or less that of 10-3L or 10-4F, thus revealing that 10-3L or 10-4F used alone has a high ability to form amyloid fibrils, and 10-3F used alone has a low ability.

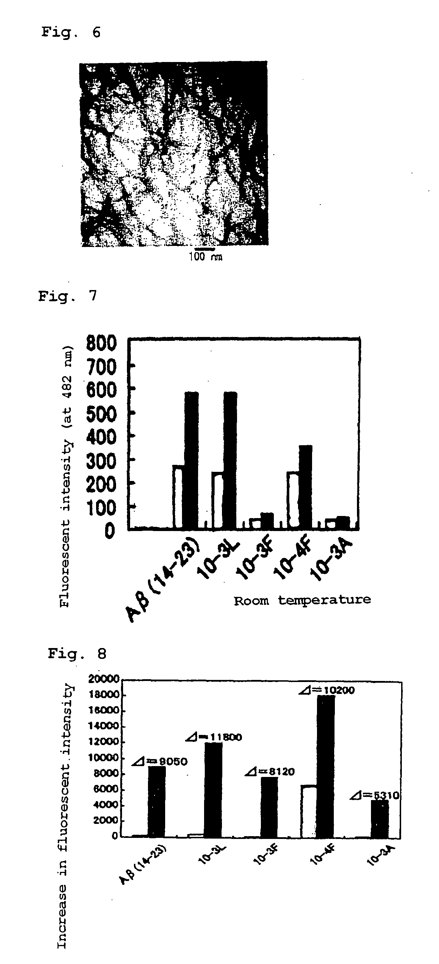

Comparison before and after the increase in fluorescent intensity of each peptide is shown in Fig. 4.

### (ii) Case where incubation was carried out at 40°C

The formation of fibrils was slow at ordinary temperatures to make it difficult to monitor the change with time, so the ability of each artificial peptide used alone to form fibrils was then evaluated at an elevated temperature of 40°C estimated to accelerate formation of fibrils.

The time required for Aβ (14-23) or 10-3L to finish formation of fibrils was significantly shortened, while the time required for 10-3F or 10-4F to finish formation of fibrils was hardly changed. 10-3A hardly showed an increase in influorescent intensity. The fluorescent intensity upon completion of fibril formation was reduced as a whole.

The comparison of fluorescent intensity among the artificial peptides after completion of the fluorescent intensity increase is shown in Fig. 5.

When these samples after completion of the fluorescent intensity change were observed under TEM, fibrils were observed in 10-3L and 10-4 F (Fig. 6) . Thus, 10-3L and 10-4 F were confirmed to form fibrils.

The above results can be summarized as follows: It is revealed that under almost all conditions, 10-3L, 10-3F and 10-4F exhibit an increase in fluorescent intensity, which is particularly significant in the case of 10-3L and 10-4F. Thus, it is suggested that 10-3L and 10-4F have a high ability to form amyloid fibrils.

10-3A into which Ala poor in hydrophobicity had been introduced showed a spectrum indicative of random coil and did not show an increase in fluorescent intensity with ThT, thus suggesting that the hydrophobicity in the peptide chain is important for formation of β-sheet structure and for formation of amyloid fibrils.

### (2) Amplification, with the artificial peptide, of formation of fibrils of Aβ (10-35) formed into fibrils (monitoring, with ThT, of amplification of fibril formation)

The amplification of formation of fibrils of Aβ (10-35) formed into amyloid fibrils was attempted by using each of the artificial peptides synthesized in Example 1. A small amount of Aβ (10-35) previously formed into fibrils under the condition in (1-2) above was added to a solution of the artificial peptide, and the amplification of formation of fibrils was monitored with ThT.

### Incubation conditions

- Starting solution used: [peptide] = 2 mM solution in TFE
- Concentrations: [peptide] = 200µM, [Aβ (10-35) (nucleus)] = 10 µM
   (20 mM Tris-HCl buffer (pH 7.4))
   *Aβ (10-35) (nucleus) was homogenized by sonication for 1 hour before dilution.
- Temperature: room temperature

### Measurement conditions

- Concentration: [peptide] = 5 µM, [ThT] = 6 µM
   (20 mM Tris-HCl buffer (pH 7.4))
- Temperature: room temperature
- Stirring conditions: The whole volume of the reaction mixture was sufficiently stirred with a Pasteur pipette before sampling, and then sufficiently stirred in a cell with a Pasteur pipette.
- Excitation wavelength: 440 nm, detection wavelength: 482 nm.

### Results

The results are shown in Fig. 7.

As is evident from Fig. 7, the peptide 10-3L or 10-4F which when used alone, had a high ability to form amyloid fibrils showed a significant increase in fluorescent intensity in the presence of the nucleus. Particularly, 10-3L showed fluorescent intensity which was twice as high as that in the absence of the nucleus, thus suggesting its high ability to amplify the nucleus. The peptide 10-3F or 10-3A which when used alone, had a low ability to form amyloid fibrils showed no increase in fluorescent intensity in the presence or absence of the nucleus, and it was thus suggested that its ability to amplify the fibrils, as well as its ability to form fibrils, are low.

### Example 3

A test for the amplification of fibrils of Aβ (10-35) formed into amyloid fibrils by using the artificial peptide of the present invention in Example 2 was attempted using a plate reader.

### [Measurement using a plate reader]

### Incubation conditions

- Starting solution used: [peptide] = 5 mM solution in DMSO
- Concentrations: [peptide] = 20 µM, [Aβ (10-35) (nucleus)] = 2.5 µM
   (20 mM Tris-HCl buffer (pH 7.4))
   *Aβ (10-35) (nucleus) was homogenized by sonication for 1 hour before dilution.
- Temperature: 40°C

### Measurement conditions

- Concentrations: [peptide] = 18 µM, [ThT] = 25 µM (20 mM Tris-HCl buffer (pH 7.4))
- Sample volume: 200 µl
- Temperature: 30°C
- Stirring conditions: The whole volume of the reaction mixture was stirred 3 times for 2 seconds with a vortex and then (100 µlx2) was stirred with a micropipette in a well before measurement.
- Excitation wavelength: 440 nm, detection wavelength: 490 nm.
- Excitation power: 30000.

### Results

The results are shown in Fig. 8.

Any artificial peptide of the present invention increased fluorescent intensity in the presence of the nucleus, and the increase in fluorescent intensity by any peptide was finished in about 30 hours. In the absence of the nucleus, only 10-4F exhibited an increase in fluorescent intensity, and the other peptides did not show an increase in fluorescent intensity. From this result, it was revealed that all of the artificial peptides according to the present invention have an ability to amplify fibrils, and succeeded in amplification of Aβ (10-35) formed into amyloid fibrils.

In the presence or absence of the nucleus, the increase in fluorescent intensity before and after fibril amplification is shown in a bar graph (Fig. 8) . Among the peptides to which the nucleus had been added, 10-4F showed the largest increase in fluorescent intensity, and the high to low increase in fluorescence intensity was shown in the order of 10-4F, 10-3L, 10-3F, and 10-3A. In the peptides to which the nucleus was added and not added, the difference in fluorescence intensity after completion of fluorescent intensity change between the peptide to which the nucleus was added and the same peptide to which the nucleus was not added was highest in the case of 10-3L, and the high to low difference therebetween was shown in the order of 10-3L, 10-4F, 10-3F, and 10-3A. The peptide which shows a greater difference in fluorescent intensity, depending on the presence or absence of the nucleus, can be considered more easily applicable to the detection system, and the high to low ability to amplify fibrils in such application is in the order of 10-3L, 10-4F, 10-3F and 10-3A.

### Industrial Applicability

The artificial peptide of the present invention is useful as a reagent for amplifying the nucleus of amyloid fibrils occurring in the living body, whereby the undetectable causative protein can be amplified to a detectable level in a stage of omen. By using this reagent, diseases attributable to amyloidosis, such as Alzheimer's disease and prion disease (e. g. mad cow disease and human Creutzfeldt-Jakob disease) can be detected in an early stage (or in a stage of omen), and thus the present invention can contribute significantly to this

technical field.

## Claims

1. A reagent for amplifying the amyloid fibrosis of amyloid β-protein, which comprises a peptide consisting of 14 to 23 residues of amyloid β-peptide [hereinafter, abbreviated as Aβ (14-23) or a peptide derived from the peptide by substituting all positively charged side-chain amino acids thereof with Lys and simultaneously substituting all negatively charged side-chain amino acids thereof with Glu.

2. The reagent according to claim 1, wherein the peptide derived from Aβ (14-23) by substituting all positively charged side-chain amino acids thereof with Lys and simultaneously substituting all negatively charged side-chain amino acids thereof with Glu is further subjected to substitution of one or more hydrophobic residues in the peptide chain by other hydrophobic amino acid residues.

3. The reagent according to claim 1, wherein the peptide derived from Aβ (14-23) by substituting all positively charged side-chain amino acids thereof with Lys and simultaneously substituting all negatively charged side-chain amino acids thereof with Glu is further subjected to substitution of all hydrophobic residues in the peptide chain with Leu or to substitution of all hydrophobic residues in the peptide chain, except for Phe at the position 3 or 4 from the N-terminal side of a hydrophobic site, with Leu, or to substitution of the position 3 from the N-terminal side of the hydrophobic site with Ala and all of the remaining hydrophobic residues with Leu.

4. A method of amplifying the amyloid fibrosis of amyloid β-protein, which comprises using a reagent containing a peptide [Aβ (14-23)] consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

5. The method according to claim 4, wherein the peptide derived from Aβ (14-23) by substituting all positively charged side-chain amino acids thereof with Lys and simultaneously substituting all negatively charged side-chain amino acids thereof with Glu is further subjected to substitution of one or more hydrophobic residues in the peptide chain with other hydrophobic amino acid residues.

6. The method according to claim 4, wherein the peptide derived from Aβ (14-23) by substituting all positively charged side-chain amino acids thereof with Lys and simultaneously substituting all negatively charged side-chain amino acids thereof with Glu is further subjected to substitution of all hydrophobic residues in the peptide chain with Leu or to substitution of all hydrophobic residues in the peptide chain, except for Phe at the position 3 or 4 from the N-terminal side of a hydrophobic site, by Leu, or to substitution of the position 3 from the N-terminal side of the hydrophobic site with Ala and all of the remaining hydrophobic residues with Leu.

7. A reagent for detection of disease attributable to amyloidosis, which comprises a peptide [Aβ (14-23)] consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

8. The detection reagent according to claim 7, wherein the peptide derived from Aβ (14-23) by substituting all positively charged side-chain amino acids thereof with Lys and simultaneously substituting all negatively charged side-chain amino acids thereof with Glu is further subjected to substitution of one or more hydrophobic residues in the peptide chain with other hydrophobic amino acid residues.

9. The detection reagent according to claim 7, wherein the peptide derived from Aβ (14-23) by substituting all positively charged side-chain amino acids thereof with Lys and simultaneously substituting all negatively charged side-chain amino acids thereof with Glu is further subjected to substitution of all hydrophobic residues in the peptide chain with Leu or to substitution of all hydrophobic residues in the peptide chain, except for Phe at the position 3 or 4 from the N-terminal side of a hydrophobic site, with Leu, or to substitution of the position 3 from the N-terminal side of the hydrophobic site with Ala and all of the remaining hydrophobic residues with Leu.

10. The detection reagent according to any one of claims 7 to 9, wherein the disease attributable to amyloidosis is Alzheimer' s disease.

11. A method of detecting disease attributable to amyloidosis, which comprises using a reagent containing a peptide [Aβ (14-23)] consisting of 14 to 23 residues of amyloid β-peptide or a peptide derived from the peptide by substituting all positively charged side-chain amino acids thereof with Lys and simultaneously substituting all negatively charged side-chain amino acids thereof with Glu.

12. The detection method according to claim 11, wherein the peptide derived from Aβ (14-23) by substituting all positively charged side-chain amino acids thereof with Lys and simultaneously substituting all negatively charged side-chain amino acids thereof with Glu is further subj ected to substitution of one or more hydrophobic residues in the peptide chain by other hydrophobic amino acid residues.

13. The detection method according to claim 11, wherein the peptide derived from Aβ (14-23) by substituting all positively charged side-chain amino acids thereof with Lys and simultaneously substituting all negatively charged side-chain amino acids thereof with Glu is further subjected to substitution of all hydrophobic residues in the peptide chain with Leu or to substitution of all hydrophobic residues in the peptide chain, except for Phe at the position 3 or 4 from the N-terminal side of a hydrophobic site, with Leu, or to substitution of the position 3 from the N-terminal side of the hydrophobic site with Ala and all of the remaining hydrophobic residues with Leu.

14. The detection method according to any one of claims 11 to 13, wherein the disease attributable to amyloidosis is Alzheimer's disease.

15. A peptide represented by the following general formula [1] :
R-Lys-Gln-Lys-Leu-Leu-X-Y-Leu-Glu-Glu-R' [1]
wherein R represents a hydrogen atom or an amino-protecting group, X represents Leu, Phe or Ala, Y represents Leu or Phe, and R' represents OH or NH₂.

16. The peptide according to claim 15, which is represented by the formula: R-Lys-Gln-Lys-Leu-Leu-Leu-Leu-Leu-Glu-Glu-R' wherein R and R' have the same meanings as defined above.

17. The peptide according to claim 15, which is represented by the formula: R-Lys-Gln-Lys-Leu-Leu-Leu-Phe-Leu-Glu-Glu-R' wherein R and R' have the same meanings as defined above.

18. The peptide according to claim 15, which is represented by the formula: R-Lys-Gln-Lys-Leu-Leu-Phe-Leu-Leu-Glu-Glu-R' wherein R and R' have the same meanings as defined above.

19. The peptide according to claim 15, which is represented by the formula: R-Lys-Gln-Lys-Leu-Leu-Ala-Leu-Leu-Glu-Glu-R' wherein R and R' have the same meanings as defined above.
